Europäisches Patentamt

European Patent Office

Office européen des brevets

(19)

(12) Publication number: **0 306 132**
**A1**

# EUROPEAN PATENT APPLICATION

(21) Application number: **88306273.9**

(22) Date of filing: **08.07.88**

(51) Int. Cl.⁴: **A01N 1/02 , A01N 3/00 , A61K 35/52 , A61K 35/54 , C12N 1/04**

The title of the invention has been amended (Guidelines for Examination in the EPO, A-III, 7.3).

(30) Priority: **08.07.87 GB 8716039**

(43) Date of publication of application:
**08.03.89 Bulletin 89/10**

(84) Designated Contracting States:
**AT BE CH DE ES FR GB GR IT LI LU NL SE**

(71) Applicant: **CELL SYSTEMS LTD**
**Cambridge Science Park Milton Road**
**Cambridge CB4 4FY(GB)**

(72) Inventor: **McGrath, John, Dr.**
**16791 Cynthia Lane**
**Haslett Michigan(US)**

(74) Representative: **Sheard, Andrew Gregory et al**
**Kilburn & Strode 30, John Street**
**London WC1N 2DD(GB)**

(54) **Protecting biological material against chilling injury.**

(57) Biological material, particularly cells such as animal gamete and embryo cells, comprising membrane-enclosed structures can be protected against chilling injury by reducing the intra-membrane tension or strain. The tension or strain can be reduced by partial dehydration of the cells in, for example, a hypertonic solution of a non-toxic solute; the tension or strain reduction may take place either prior to or concomotantly with chilling.

EP 0 306 132 A1

## BIOLOGICAL CHILLING PROTECTION

This invention relates to the protection of biological samples from the damaging effects of chilling. The term "chilling" as used in this specification is defined to be a temperature reduction without necessarily the formation of ice. The term "biological sample" as used in this specification includes cells (both eukaryotic and prokaryotic) and other membrane-enclosed structures, organs and tissue composed of cells, viruses, all of which can be natural or genetically or otherwise modified, by way of example. The invention finds particular application in the chilling protection of gametes and embryos, particularly porcine ova and embryos and of plant material such as crops.

The storage of cells in the frozen state (cryopreservation) is a technique commonly used for the long-term maintenance of cellular viability and genetic stability. However, a major problem for some biological samples is that the sample is damaged due to chilling at temperatures above those associated with freezing, thereby preventing successful cryopreservation.

Furthermore, it is known that chilling damage can occur in the supercooled state which suggests that the detrimental effects of temperature reduction without the formation of ice also occur at temperatures where ice is present in some cases. Therefore it is likely that the mechanisms of chilling damage continue to play a role when ice is present. As a result it is reasonable to assume that protection from chilling injury can provide protection during freezing as well.

Chilling injury is also a major problem representing a form of environmental stress imposed upon agricultural crops. Plant species of temperate origin do not generally manifest this form of injury which is in sharp contrast to many important crops of tropical or subtropical origin (rice, maize, tomato, cotton, soybean etc), which are sensitive to temperatures in the range of $20\,^\circ\mathrm{C}$ to $0\,^\circ\mathrm{C}$.

A distinction is normally made between indirect and direct forms of chilling injury. Indirect chilling injury takes longer to manifest itself (hours or days) and is independent of the rate of cooling whereas direct chilling injury is apparent soon after chilling (seconds or minutes) and the amount of injury is related to the rate of cooling. Direct chilling is also referred to as "cold shock" or "thermal shock". Indirect and direct chilling injury may represent limiting responses of a single phenomenon. Both may be controllable by means of the present invention.

It is a well-recognised fact that chilling injury to crops is an important form of environmental stress to many plants which has an enormous impact on the world food supply. Chilling injury of bacteria is also common and is important both with respect to long-term preservation and to sterilization technology.

In contrast to these cases where the importance of cold shock is accepted the importance of chilling injury to mammalian cells was thought to be restricted to a small number of unusual cell types until recently. Those cases where chilling injury was well-recognised was limited to some spermatozoa (ram, bull and stallion), bovine and murine embryos prior to the late morula stage, procine embryos at all stages of development and human polymorphonuclear cells.

However recent experimental work and a review of the literature suggests that chilling injury may be very widespread indeed. The cell types where chilling injury has been demonstrated now include: a) bacteria; b) yeasts; c) protozoa; d) algae; e) higher plants; f) mammalian cells (spermatozoa, embryos, isolated hepatocytes, lymphoid cell line, granulocytes and erythrocytes).

What is more it has been argued that the mechanisms responsible for chilling injury do not disappear once freezing commences. Thus the protective effects described here are expected to be in place for temperatures below $0\,^\circ\mathrm{C}$ as well as above $0\,^\circ\mathrm{C}$.

The underlying cause of chilling injury damage is not well understood. However, it is generally accepted that chilling injury is associated with damage to cellular membranes. The most widely-accepted theory is that of Lyons and Raison. This theory explains chilling injury in terms of a temperature-induced phase transition of lipids from gel to liquid crystal in cellular membranes and/or a temperature-dependent alteration in the hydrophobic nature of membrane proteins. "This change of state would be expected to bring about a contraction that causes cracks or channels leading to increased permeability". Other explanations of chilling injury to membranes include: a) increased membrane permeability due to the formation of defects at the interface between solid and liquid portions of membrane and b) disruption of membrane-bound enzyme function due to phase separation effects in the membrane.

This theory explaining the mechanism of chilling damage has emerged from the study of plant chilling injury and has been applied to virtually all other cell types. It is not without problems however. A very serious problem is that there are numerous examples where chilling injury occurs but no membrane phase changes can be detected. A recent symposium was convened to critically assess the theory. The conclusions were summarised as a list of points many of which are in direct contradiction with this popular theory.

As a result this theory does not provide a fundamental basis at the present time for designing procedures to prevent chilling injury other than to say that for those systems where a membrane phase transition is detected, any proposed method must reduce the possibility of producing damaging processes associated with the phase transition.

The present invention rests on a completely different theory: the thermoelastic behaviour of biological membranes forms the basis of the invention claimed here. Very simply summarized, damage to membranes will arise when they are chilled because the membrane material has a tendency to contract more than the liquid contained within the membrane-bound system. As a result there arise situations where membranes squeeze internal liquid thereby developing a stress or strain in the membrane. The stress or strain levels involved can lyse membranes. In the general case any type or number of cellular membrane may be at risk in a given case. In some cases the plasmalemma may be most suceptible. In others the membranes of one or more organelles may be most sensitive. As a general rule structures with flaccid membranes will be less susceptible to any chilling injury produced by thermoelastic stress or strain. It is important to make a distinction between the popular phase-transition based hypothesis of chilling injury and the thermoelastic theory. As its name implies the former requires a phase transition or phase separation event in the membrane to induce injury. From the viewpoint of the thermoelastic theory membrane injury due to chilling can occur with or without the occurrence of a phase event in the membrane.

According to a first aspect of the present invention, there is provided a method of protecting biological material comprising membrane-enclosed structures against chilling injury, the method comprising reducing the intra-membrane tension or strain.

The biological material may comprise a prokaryotic or (preferably) eukaryotic cell, particularly an animal or plant cell. Gametes and embryos are cells which the invention may be particularly appropriate at protecting, particularly from commercially important animals such as pigs, sheep, cows and other livestock.

The invention involves the production of flaccid membranes, for example in all of the membrane-bound structures of the cells involved in the system prior to or during chilling. The term "flaccid membrane" is defined here to be a membrane in a virtual tension-free state with "excess" membrane surface area. The tension-free state is defined as an in-plane tension level insignificantly small compared with the tension level required for lysis. Excess membrane surface area is defined in relation to the surface area necessary to enclose the volume of the membrane-bound structure in a sphere. Since the surface area to volume ratio is a minimum for a spherical geometry all additional area is defined to be excess in this sense.

There are a variety of ways in which the membrane strain or stress may be reduced, and the membrane may be made to be relatively flaccid. One preferred method involves producing flaccid membranes by removing water from the system, for example by osmosis. Such dehydration in effect produces "excess" membrane surface area in the form of folds, pleats etc. Contraction caused by cooling will retract this area prior to the development of critical tension in the membranes. The hypertonic solution will generally have a concentration of solute of at least 0.3M, 0.35M or 0.4M. In general more protection will arise from increasingly hypertonic solutions because of additional shrinkage. However, too high a concentration of solute may have a toxic or other deleterious effect. It is therefore generally preferred to keep the concentration of the solute in the hypertonic solution below 1.5M, 1.2M or 1.1M. 1.1M is a generally preferred maximum value. Since a distribution of membrane surface area-to-volume ratios is expected in a population of cells and organelles it will be important to provide enough shrinkage for those structures with the smallest initial area-to-volume ratios.

Osmotic dehydration has been shown to produce an irreversible loss of membrane material in some instances. As a consequence membrane-bound systems such as cells and internal organelles which experience such loss may be expected to lyse upon rehydration because they have insufficient membrane area to expand back to the initial volume. In the present embodiment of the invention, membrane-bound structures will generally be dehydrated to an extent, for example the maximum extent, that still enables reversible rehydration. Such a procedure will minimize the likelihood of developing lytic thermoelastic membrane stress during chilling. Operationally, reversible rehydration can always be defined by subjecting the biological system of interest to a dehydration/rehydration cycle without chilling followed by a suitable recovery assay. The "suitable" recovery assay will depend upon how chilling injury is defined. Examples include cell division, pregnancy, motility, exclusion of vital dyes, leakage of molecules, etc. A series of dehydration/rehydration tests spanning a range of maximum extents of dehydration will establish osmotic tolerance limits.

Chilling protection by a method in accordance with the present invention may be assessed by applying the recovery assay after the following sequence: dehydration, chilling, warming, rehydration. The range of acceptable extents of dehydration to be applied are defined by the osmotic limit tests described above.

3

When recovery is enhanced compared to samples chilled and warmed without dehydration and subsequent rehydration chilling protection has been realized by the invention.

It is usually important that the solutes used to produce the dehydration be non-permeating or only slowly permeating and non-toxic. Examples of such solutes include, in many instances: saccharides (e.g. sucrose, trehalose), amino acids (e.g. proline), betaines (e.g. glycine, betaine) and polyols such as glycerol.

The biological material should not be kept for too long in hypertonic solution in case portions of the membrane 'bleb off' in separate particles.

Dehydration produces flaccid membranes; temperature reduction contracts membranes. As long as the extent of dehydration compensates for the contraction produced by the temperature drop lytic membrane tensions will not be developed. As a consequence both static and dynamic methods of achieving this compensation will be effective. A static method is defined here to be a case where the biological system is dehydrated (or otherwise stress/strain-reduced) to an equilibrium extent prior to chilling. An example of this method would be exposing an embryo to a non-toxic, non-penetrating solute such as trehalose. The embryo or other biological material would be allowed to shrink to an equilibrium minimum volume and then cooling would be initiated.

A dynamic method is defined here as the case where the biological system is not in osmotic equilibrium while the temperature is changing. A simple example of this type of method consists of exposing an embryo to a penetrating solute such as glycerol. The embryo is expected to shrink (rather rapidly) initially as water leaves the cells but to swell again (rather slowly) as glycerol enters the cells. Chilling protection will be imposed if cooling occurs while the embryo is shrunken. A more complex dynamic method would consist of simultaneously varying the concentration of a non-penetrating extracellular solute and the temperature in order to keep membranes flaccid at all times. An example of this type of procedure would be repetition of an incremental increase in extracellular concentration followed by an incremental decrease in temperature, thereby slackening membranes in steps sufficiently to compensate for the subsequent contraction by a cooling step.

Although dehydration may be the preferred way of reducing membrane strain or stress, other methods may be used. For example, membrane area could be increased by fusion with liposomes or other appropriate structures.

The stress or strain of a membrane may be measured or at least indicated by a variety of techniques. for example those involving membrane probes such as chemical fluorescent probes. Other molecules whose optical or other characteristics vary with their membrane environments may also be used.

The invention rests on the emerging field of biological membrane mechanics. Much of the current state-of-the-art in this field is summarized in a recent book by Evans and Skalak. This book and related literature define a theoretical basis for describing the mechanics and thermodynamics of biological membranes including theory relevant to the development of membrane stresses due to temperature reduction. The theory described in these references is not developed in any way to explain how chilling injury may occur in biological membranes. A more recent publication has shown that temperature reduction can result in lysis of cell-size lipid vesicles (liposomes). The fact that lysis occurred for temperature reductions which could be predicted on the basis of the development of thermoelastic stress in the vesicle membrane suggests that this is the cause of lysis. However, no mention is made in this publication of the possible relevance of this phenomenon to biological chilling injury nor is any means suggested to prevent biological chilling injury.

McGrath has expanded the thermoelastic theory described above - most importantly by including dynamic effects and has developed computer programs to predict the response of model membranes to various chilling procedures. The data base defining the biophysical parameters needed to use the theory are scarce. However the prediction appear to be consistent with every known major feature of chilling injury.

The theory is based on a model which outlines the relationship between membrane biophysical parameters and external factors which may mediate membrane response. In addition to the fact that the theory correctly accounts qualitatively for all known features of chilling injury it can be used to define methods to manipulate biophysical properties of the membrane and external factors in order to reduce or eliminate chilling injury.

In this connection the theory has suggested that internal organelles rather than the plasmalemma are more likely to be primary targets of chilling injury. This prediction has been substantiated by experimental observation but the reason for these structures being the likely primary targets has not been appreciated:

"The reason for the tonoplast lesion per se under chilling remains unsolved".

Furthermore, the inventor's own experimental evidence using a unique computer-controlled light microscopic heat transfer stage has provided graphic visual evidence of lesions in chilled isolated plant vacuoles for conditions predicted by theory. Vacuoles from chilling-resistant plants required larger temperature drops to induce lysis as compared to vacuoles isolated from chilling-sensitive plants.

There is no previous systematic use of the concepts in this theory to minimize or eliminate chilling injury by manipulating membrane thermoelastic properties or external factors relevant to the theory.

Many methods have been applied to ameliorate chilling injury. These methods include: reducing cooling rate; temperature conditioning; alternating temperatures; hyperbaric storage; modified atmospheres; exposure to common penetrating cryoprotective compounds; exposure to various ions, phospholipids, sterols, alcohols, chelators, sugars, lipoproteins and drugs as a means of exerting a stabilizing effect directly on the membrane; drought-hardening of plants and other treatments. None of these treatments have been linked to the concept of producing reversibly flaccid cell membranes by for example dehydration as means of avoiding lytic thermoelastic stress in membranes.

It is expected on the basis of the thermoelastic stress theory that chemicals may be used to exert a protective effect directly on the membrane to avoid chilling injury. These compounds should have the following effects on the membranes if they are to exert their protective effect:

a) Increase maximum tolerable membrane strain or stress limits which can be sustained prior to lysis;

b) Decrease the stiffness (elastic modulus) of membranes;

c) Decrease the tendency of membranes to contact with temperature reduction (thermal expansitivy coefficient); and/or

d) Increase the membrane water permeability.

Genetic engineering techniques may be applied to effect these changes in cellular membranes as a means of reducing or preventing chilling injury; the present invention includes such genetically engineered cells, which may include appropriately modified proteins or glycoproteins in their membrane(s).

For cell-size membrane-bound structures the first three characteristics are directly testable using micropipette aspiration techiniques. The fourth may be determined by several methods including direct observation or by light scattering.

It is by no means clear or predictable from the existing literature that dehydration should protect against chilling injury. Leder has shown that the application of a hyperosmotic solution at the moment of chilling can prevent the loss of permease accumulated substrates. Viability was not measured and no claim was made that such hyperosmotic treatment could be used to prevent the loss of viability during chilling. Strange has cited results which suggest that a lower extracellular osmolarity (less dehydration) is less likely to result in chilling injury. Similarly Meynell presented results which show no clear effect of solution osmolarity. The plant data mentioned earlier reveal no clear and consistent effect of dehydration.

There is in fact evidence in the literature to suggest that exposing cells to concentrated solutions may predispose the cell to chilling injury. One of the cell types most extensively studied with respect to cold shock is the human erythrocyte (red blood cell).

For this cell type the cell is not chilling sensitive for normal (isotonic) solute concentrations. In contrast, exposure to hypertonic solutions can cause significantly more damage due to chilling. This is a special case because many other cells do not become sensitized to chilling when exposed to hypertonic solutions. Nevertheless, this evidence is in direct opposition to the method proposed here in as much as it suggests that cellular dehydration is to be avoided rather than imposed in order to reduce chilling injury.

In the field of cryopreservation it is known that dehydration during freezing is actually beneficial in some cases primarily as a means of avoiding cell damage accompanying the formation of intracellular ice. As chilling injury is normally defined to occur in the absence of freezing there would be no apparent reason on this basis to dehydrate cells to avoid chilling injury since ice is not assumed to be present. Therefore the benefits of dehydration in relation to freezing would not be directly applicable to chilling injury.

The invention will now be illustrated by the following examples.

Example 1.

Chilling Protection of Porcine Embryos.

### 1A. Sample Isolation/Preparation

Embryos were collected surgically from mated sows. Embryos at two. four and eight cell stage of development were collected in medium with 20% sheep serum. Embyros were transferred to PBS within 6 hours and treated within 12 hours after collection. Embryos were maintained at or near room temperature (20° C) prior to treatment.

### 1B. Damage Assay

Viability was assayed by flourescence assay. The embryos were challenged with a fluorescent dye technique commonly used for such purposes some 24 hours after treatment. A stock solution of first dye was diluted into the petri dish containing 5 ml of PBS to an appropriate final concentration. A stock solution of second dye was diluted into the petri dish to its final concentration. Embryos were exposed at room temperature to these dyes for a given number of minutes and then observed and photographed using a light microscope set up for fluorescence. This fluorescence assay is known for embryos to have a high degree of correlation with more vigorous means of testing viability.

### 1C. Control 1: Untreated, Unchilled

No-treatment control consisted of placing the embryos in 5 ml of PBS in petri dishes kept at room temperature throughout.

### 1D. Control 2: Untreated Chilled

Controls were also performed for chilling without treatment in cases where damage has been consistently reported in the literature. An embryo in 5 ml of PBS in a petri dish was placed directly in the Fryka constant temperature bath and cooled. The petri dish was removed and allowed to warm on the laboratory bench.

### 1E. Dehydration/Rehydration Treatment

The non-chilled osmotically-treated embryos were treated as the chilled embryos with the exception that they were held at room temperature throughout. Individual embryos were placed into hypertonic solutions at room temperature and allowed to equilibrate osmotically by dehydrating by exposure for a period of 5 minutes. Individual embryos were removed from the hypertonic solution and placed into the initial isotonic solution at room temperature.

### 1F. Treated. Chilled

Individual embryos were removed from the PBS solution by micropipette and placed in 5 ml of the solution of interest contained in a plastic petri dish at room temperature (20° C). The embryos were allowed to equilibrate in the solution of interest for a number of minutes.

The petri dish containing the embryo was then immersed into a constant temperature bath (Fryka) with a given set point temperature. A duplicate sample containing a copper-constantan thermocouple but no embryo was immersed into the bath at the same time to display the temperature history of the sample on digital temperature meter. When the duplicate sample temperature reached a given temperature the petri dish containing the embryo was removed immediately, wiped dry and placed upon the laboratory bench. The cooling rate was noted.

The embryo was removed by micropipette and placed into a petri dish containing 5 ml of PBS solution at room temperature.

1G. Results

Results as determined by the fluorescence assay are shown in Table 1. These results show that there is no loss of viability in untreated, unchilled embryos. Untreated, chilled embryos show no viability which confirms previous data. The dehydration/rehydration experiments show that at room temperature embryos tolerate one-step dehydration/rehydration osmotic excursions from isotonic medium up to isotonic medium plus 1.0 M trehalose and back to isotonic medium. Finally the data show that dehydrated embryos can be chilled, rewarmed and rehydrated with approximately 50% recovery indicating prevention of chilling injury.

Table 1

| Fluorescence Assay | |
| --- | --- |
| 1. Control | % Viability |
| Untreated Unchilled | . 100% |
| 2. Control | |
| Treated Unchilled | 100% |
| 3. Control | |
| Untreated Chilled | 0% |
| 4. Treated and chilled | 50% |

Example 2.

Chilling Protection of Porcine Embryos.

The procedure of Example 1 was followed, except that the trehalose concentration was reduced to 0.5M, and viability was assessed by regrowth in vitro. Embryos were transferred to glass vials containing medium with 20% sheep serum, sealed and incubated at 37°c in a water bath. After 48 hours the embryos were examined microscopically; those blastocytes which had hatched from the zone pelucida were considered viable. Results are shown in Table 2.

EP 0 306 132 A1

Table 2

| Regrowth in vitro | |
| --- | --- |
| 1. Control | % Viability |
| Untreated Unchilled | 82% |
| 2. Control | |
| Treated Unchilled | 66% |
| 3. Control | |
| Untreated Chilled | 0% |
| 4. Treated and chilled | 61% |

Example 3.

Chilling Protection of Porcine Embryos.

The procedure of Example 1 was followed, except that the trehalose concentration was reduced to 0.5M, and viability was assessed by pregnancy. Embryos were surgically transferred into recipient sows. Those sows not returning to heat after 21 days were examined surgically for the presence of foetuses. On two occassions embryos were chilled and then transferred into respective recipient sows. Neither sow returned to heat; 21 days afterwards, normal foetuses had implanted.

Example 4. Chilling Protection of Ram spermatozoa.

4A. Sample Collection

Samples were collected conventionally.

4B. Damage Assay

Spermatozoa motility was taken as a measure of viability. Following treatment samples were incubated at $37°$ C for a number of hours prior to assay.

4C. Control 1: Untreated, Unchilled Samples

Spermatozoa in test tubes were kept at a constant temperature for periods of time equal to that required for chilling experiments.

Following this period samples were removed from the test tube and examined using a light mircoscope to determine sperm motility.

8

### 4D. Control 2: Untreated, Chilled Samples

Spermatozoa in test tubes initially at 30°C were chilled to 0°C by placing test tubes containing spermatozoa into a constant temperature bath held at 0°C prior to warming.

The samples were assayed for viability as described above.

### 4E. Test: Dehydration/Rehydration Tests.

An appropriate dehydration/rehydration test was carried out.

### 4F. Test: Treated, Chilled Samples

Spermatozoa were placed in 5ml test tubes containing 4ml of 1.5M trehalose in PBS and allowed to equilibrate at for 10 minutes. The test tubes were warmed and assayed as described in Part D for the untreated, chilled control samples.

### Example 5.

### Chilling Protection of Bacteria: E coli.

### 5A. Materials and Methods

Bacterial strains The bacteria were E coli K-12.

Medium. Cells were grown in medium 63 (16) containing thiamine (0.5mg/litre) and glycerol (0.2%). Medium 63 is composed of $0.1M$ $KH_2PO_4$, $0.015M$ $(NH_4)_2SO_4$. $0.8mM$ $MgSO_4$, $1.8 M$ $FeSO_4$. The pH was adjusted to 7.0 and 7.2 with KOH. The osmolarity is approximately 0.3. For strain B34-5, this medium was supplemented with 0.2% Casamino Acids and designated medium 53C.

### Growth and preparation for permease determination.

Bacteria were grown overnight to cell densities of $3 \times 10$ to $8 \times 10^8$ cells per ml. lac permease was induced by 0.5mM IPTG and TNG permease II by 1 mM galactose (13).

The bacteria were collected by centrifugation, resuspended in fresh medium in the absence of an inducer, and after an additional incubation period of approximately 20 min centrifuged and suspended in medium 53C at a cell density of $1.8 \times 10^8$ cells per ml, The cell suspension was kept in an ice bath throughout a day's experiments.

### 5B. Damage Assay

The cells were assayed for damage appropriately.

### 5C. Control 1: Untreated, Unchilled

An appropriate control was undertaken.

### 5D. Control 2: Untreated, Chilled

A sample chilled from 25°C to 0°C at osmolality of 0.3 in 1 to 2 secs (500 - 1000 C/min) results in 95% damage: if cooled over a period of 1 min 25 C/min 0% damage results.

### 5E. Dehydration/Rehydration

A sample was taken from 0.3 osm to 0.6 osm and back at a constant temperature and assayed.

### 5F. Treated, Chilled

A sample was taken from 0.3 osm to 0,6 osm at a given temperature, chilled at 500-1000 C/min from 25°C to 0°C, rewarmed, rehydrated and assayed.

### Example 6

### Chilling Protection of a protozoan: Tetrahymena pyriformis

### 6A. Sample Preparation

Cell culture. T. pyriformis (Culture Centre of Algae and Protozoa, Cambridge, strain CCAP 1630/1W) was grown axenically in PY medium (1.0% proteose peptone, 0.25% yeast extract), and unless otherwise stated, cells were from cultures maintained for 3 days at 20°C. Under these conditions the cultures were in the early stationary phase of growth. Modifications of growth medium and culture conditions were made as necessary.

### 6B. Damage Assay

Cellular viability was determined by a most probable number assay using microtiter plates. There were at least four replicates for each treatment.

### 6C. Control 1: Untreated, Unchilled

An appropriate control was undertaken.

### 6D. Control 2: Untreated, Chilled

Chilling and Rewarming. Aliquots of cell suspension (0.25 ml) were cooled in polypropylene tubes (Eppendorf) to 0°C. An "instantaneous" rate of cooling was obtained by rapidly pipetting 0.1 ml of cell suspension into 9.9 ml of precooled growth medium, and the intial step for control cells was an equivalent dilution at 20°C. Cells were rewarmed to 20°C at a rate of 70°C.min$^{-1}$.
All cell suspensions in which ice had formed were discarded.

### 6E. Dehydration/Rehydration Treatment

An appropriate dehydration/rehydration routine was established.

6F. Treated, Chilled

The cells were treated in accordance with the invention.

Claims

1. A method of protecting biological material comprising membrane-enclosed structures against chilling injury, the method comprising reducing the intra-membrane tension or strain.

2. A method as claimed in claim 1, wherein the biological material comprises one or more prokaryotic or eukaryotic cells.

3. A method as claimed in claim 1, wherein the biological material comprises a gamete or embryo.

4. A method as claimed in claim 1, 2 or 3, wherein the intra-membrane tension or strain is reduced by partial dehydration.

5. A method as claimed in claim 4, wherein the partial dehydration is achieved by osmosis.

6. A method as claimed in claim 5, wherein the osmosis is achieved by use of a hypertonic solution of a saccharide, an amino acid, a betaine or a polyol.

7. A method as claimed in any one of claims 1 to 5, wherein the intra-membrane tension or strain is reduced prior to chilling.

8. A method as claimed in claim 1, 2 or 3, wherein the intra-membrane tension or strain is reduced by increasing the surfsce area of the membrane.

9. A method of protecting biological material comprising membrane-enclosed structures against chilling injury, the method comprising contacting the biological material with a compound which has at least one of the following effects:

a) Increase maximum tolerable membrane strain or stress limits which can be sustained prior to lysis;

b) Decrease the stiffness (elastic modulus) of membranes;

c) Decrease the tendency of membranes to contact with temperature reduction (thermal expansitivy coefficient); and/or

d) Increase the membrane water permeability.

10. Biological material having a membrane incorporating a recombinant (glyco)protein which has at least one of the following effects:

a) Increase maximum tolerable membrane strain or stress limits which can be sustained prior to lysis;

b) Decrease the stiffness (elastic modulus) of membranes;

c) Decrease the tendency of membranes to contact with temperature reduction (thermal expansitivy coefficient); and/or

d) Increase the membrane water permeability.

European Patent Office

# EUROPEAN SEARCH REPORT

Application Number

EP 88 30 6273

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | CHEMICAL ABSTRACTS, vol. 90, no. 3, 15th January 1979, page 400, no. 20038e, Columbus, Ohio, US; J.B. GRIFFITHS: "Effect of hypertonic stress on mammalian cell lines and its relevance to freeze-thaw injury", & CRYOBIOLOGY 1978, 15(5), 517-29 | 1-10 | A 01 N 1/02<br>A 01 N 3/00<br>A 61 K 35/52<br>A 61 K 35/54<br>C 12 N 1/04 |
| A | CHEMICAL ABSTRACTS, vol. 74, no. 15, 12th April 1971, page 216, no. 73856w, Columbus, Ohio, US; H.T. MERYMAN: "Exceeding a minimum tolerable cell volume in hypertonic suspension as a cause of freezing injury", & FROZEN CELL. SYMP. 1969 (PUB. 1970), 51-67 | 1-10 | |
| A | CHEMICAL ABSTRACTS, vol. 77, no. 7, 14th August 1972, page 302, no. 46249f, Columbus, Ohio, US; J. FARRANT et al.: "Human red cells under hypertonic conditions. Model system for investigating freezing damage. 2. Sucrose", & CRYOBIOLOGY 1972, 9(1), 16-21 | 1-10 | TECHNICAL FIELDS SEARCHED (Int. Cl.4)<br><br>A 01 N<br>A 61 K<br>C 12 N |
| A | CHEMICAL ABSTRACTS, vol. 105, no. 17, 27th October 1986, page 290, no. 148508d, Columbus, Ohio, US; D.K. HINCHA: "Sucrose influx and mechanical damage by osmotic stress to thylakoid membranes during an in vitro freeze-thaw cycle", & BIOCHIM. BIOPHYS. ACTA 1986, 861(1), 152-8 | 1-10 | |
| A | US-A-4 155 331 (A.L. LAWRENCE et al.)<br>* Column 3, line 16 - column 4, line 27; column 5, lines 10-50; claims * | 1-10 | |

-/-

The present search report has been drawn up for all claims

| Place of search | Date of completion of the search | Examiner |
|---|---|---|
| THE HAGUE | 25-10-1988 | FLETCHER A.S. |

European Patent
Office

EUROPEAN SEARCH REPORT

Application Number

EP  88 30 6273

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | WO-A-8 402 389  (J.J. McKENNA)<br>* Page 3, lines 5-11; page 4, line 29 - page 5, column 22; page 7, line 37 - page 8, line 15 * | 1-3 | |

TECHNICAL FIELDS
SEARCHED (Int. Cl.4)

The present search report has been drawn up for all claims

| Place of search | Date of completion of the search | Examiner |
|---|---|---|
| THE HAGUE | 25-10-1988 | FLETCHER A.S. |

CATEGORY OF CITED DOCUMENTS

X : particularly relevant if taken alone
Y : particularly relevant if combined with another
      document of the same category
A : technological background
O : non-written disclosure
P : intermediate document

T : theory or principle underlying the invention
E : earlier patent document, but published on, or
      after the filing date
D : document cited in the application
L : document cited for other reasons
...........................................................................................
& : member of the same patent family, corresponding
      document

EPO FORM 1503 03.82 (P0401)